# EUROPÄISCHE PATENTANMELDUNG

(11) **EP 0 982 034 A1**
(43) Veröffentlichungstag der Anmeldung: **01.03.2000**
(21) Anmeldenummer: 99116722.2
(22) Anmeldetag: 26.08.1999
(51) Int. Cl.: A61K 35/56, A61K 31/7008

(54) **Zusammensetzung enthaltend Glucosamin und einen Extrakt aus Perna canaliculus**

(30) Priorität: 26.08.1998 DE 19838794
(71) Anmelder: aristavet Veterinärspezialitäten GmbH & Co. KG, 88212 Ravensburg (DE)
(72) Erfinder: Wahle, Helge, Dr., 88212 Ravensburg (DE)
(74) Vertreter: HOFFMANN - EITLE

(57) **Zusammenfassung**

Die Erfindung betrifft eine Zusammensetzung, umfassend einen Muschelextrakt und eine Glucosamin-Komponente, ein Diätetikum umfassend diese Zusammensetzung, ein pharmazeutisches Präparat umfassend diese Zusammensetzung sowie die Verwendung der Zusammensetzung zur nutritiven Versorgung des Gelenkknorpels und zur Behandlung von Osteoarthritis beim Menschen wie auch beim Tier. Der Muschelextrakt wird aus der Muschel *Perna canaliculus* gewonnen.

## Beschreibung

Die Erfindung betrifft eine Zusammensetzung, umfassend einen Muschelextrakt und eine Glucosamin-Komponente, ein Diätetikum (Nahrungsergänzungsmittel, Ergänzungsfuttermittel) umfassend diese Zusammensetzung,ein pharmazeutisches Präparat umfassend diese Zusammensetzung sowie die Verwendung der Zusammensetzung zur nutritiven Versorgung des Gelenkknorpels und zur Behandlung von Osteoarthritis beim Menschen wie auch beim Tier.

Degenerative Gelenkerkrankungen wie Osteoarthritis sind in der Bevölkerung und bei Nutz- und Heimtieren sehr weit verbreitet. Sie führen sowohl beim Menschen als auch bei Tieren, insbesondere bei Hund und Pferd, zu intermittierenden oder chronischen Schmerzen, zu einer Verminderung der Gelenksbeweglichkeit, intermittierenden oder chronischen Lahmheiten und zu entzündlichen Veränderungen im Gelenk.

Die Osteoarthritis ist eine Erkrankung des Gelenkknorpels. Gelenkknorpel besteht aus Chondrozyten, einer Interzellularsubstanz aus Kollagenfasern und einer gut hydrierten Grundsubstanz, die von Proteoglykanen und Glykoproteinen gebildet wird. Die Chondrozyten produzieren eine Interzellularsubstanz, die für die Gelenktunktion Festigkeit, Gleitfähigkeit, Dauerhaftigkeit und Belastungsausgleich geeignet ist.

In der Grundsubstanz befinden sich die Proteoglykane, die den Raum zwischen den Kollagenfasern ausfüllen. Sie können verschiedene Formen annehmen und bilden Strukturen, die aus einem Proteinherz und Seitenketten aus Glykosaminoglykanen (GAG; früher auch als Mucopolysaccharide bezeichnet) bestehen. Die meisten bilden mit den Hyaluronsäurefasern in nicht-kovalenten Bindungen große Aggregate. Die Bindung zwischen dem Proteinherzen aus Proteoglykanen und dem Hyaluronmolekül wird durch ein Verbindungsprotein stabilisiert. Die Bildung von Proteoglykan-Aggregaten verhindert vermutlich den Verlust von Monomeren aus der Interzellularsubstanz, stabilisiert die Kollagenfasern und kontrolliert den Wasserzu- und -abfluß des Knorpelgewebes. Die Seitenketten aus GAG aus den Proteoglykanen sind nicht alle gleich. Hauptsächlich kommen die drei Seitenkettentypen Chondroitin-6-sulfat, Chondroitin-4-sulfat und Keratansulfat vor. Alle drei sind lineare Polymere, die aus sich wiederholenden Disacchariden bestehen. Die Disaccharid-Einheiten besitzen ein oder zwei negativ geladene Gruppen, die, wenn sie in Ketten von 50-70 Dimereneinheiten enthalten sind, stark abstoßende Ketten bilden, die steif vom Proteinherz abstehen. Die negativen Ladungen ziehen eine Hydratation nach sich, was dem Knorpelgewebe Steifheit und Kompressibilität verleiht. Wenn das Knorpelgewebe komprimiert wird, werden die Proteoglykan-Aggregate zusammengedrückt und Wasser aus dem Knorpelgewebe gepreßt. Sobald der Druck nachläßt, kann das Wasser zurückfließen, und die Proteoglykanmonomere nehmen ihr ursprüngliches Volumen wieder ein. Unter physiologischer Belastung kann das Knorpelgewebe auf bis zu 40 Prozent seiner ursprünglichen Höhe zusammengepreßt werden (Equine vet. Educ. (1991); 3(2); 72-75).

Die Chondrozyten synthetisieren alle Bestandteile der Grundsubstanz und sind für die Erhaltung der Matrix und ihren Turn-Over verantwortlich. Der konstante Syntheseprozeß erfordert große Mengen an Bausteinen für die Synthese der Kollagenfasern und Proteoglykane. Wenn die für diese Bausteine benötigten Nährstoffe nicht in ausreichender Menge zur Verfügung stehen, wird der Syntheseprozeß beeinträchtigt und der Knorpel verliert seine Eigenschaft, sich selbst zu regenerieren.

Osteoarthritis resultiert aus einer nutritiven Unterversorgung mit Bausteinen der Proteoglykane, also einem Ungleichgewicht zwischen der Syntheserate und dem Verlust an Proteoglykanen (Medical Hypotheses (1994), 42; 323-327).

Es sind bereits medikamentöse Behandlungen der Osteoarthritis bekannt. Die medikamentöse Standardtherapie besteht aus der Verabreichung von Corticosteroiden und nicht-steraidalen Antiphlogistika. Weiterhin ist die Gabe sog. Chondroprotektiva zur nutritiven Versorgung des Gelenkknorpels mit Metaboliten für die Bildung von Proteoglykanen und zur Behandlung der Osteoarthritis bekannt, wie z.B. von Glykosaminoglykanen oder Hyaluronsäure. Diese Stoffe sollen den Knorpelabbau hemmen, und durch Anregung der Proteoglykansynthese soll die Matrixsynthese der Chondrozyten verbessert werden. Weiterhin ist auch die Verwendung von Glucosamin als Chondroprotektivum bekannt.

Beispielsweise offenbart WO94/22453 eine Kombination aus Glucosamin und Chondroitinsulfat zur Therapie der Osteoarthritis.

Weiterhin ist zur Behandlung der Osteoarthritis ein Extrakt aus der Muschel *Perna canaliculus* verwendet worden.

Aufgabe der Erfindung ist es, die Wirksamkeit der im Stand der Technik erwähnten Zusammensetzungen als Chondroprotektiva und zur Behandlung von Osteoarthritis zu verbessern.

Diese Aufgabe wird erfindungsgemäß durch eine Zusammensetzung, umfassend einen Extrakt der Muschel *Perna canaliculus* und einer glucosamin-haltigen Komponente, gelöst.

Diese beiden Komponenten der erfindungsgemäßen Zusammensetzung liegen vorzugsweise in einem Gewichtsverhältnis von Glucosamin-Komponente zu Muschelextrakt in dem Bereich von 0,2 bis 6, besonders bevorzugt in dem Bereich von 0,5 bis 2,5 vor. Am bevorzugtesten ist ein Gewichtsverhältnis in dem Bereich von 0,8 bis 1,6.

Die erfindungsgemäß zu verwendende Glucosamin-Komponente enthält D-Glucosamin bzw. Derivate oder Salze, insbesondere Natrium- und Kaliumsalze, hiervon. Besonders vorteilhaft verwendet werden D-Glucosamin, D-Glucosaminsulfat, D-Glucosamin-HCl, N-Acetyl-D-Glucosamin oder eine Mischung hiervon.

Die erfindungsgemäß zu verwendende Glucosamin-Komponente kann bevorzugt aus Chitin gewonnen werden. Chitin hat durch die β-1,4-Verknüpfungen von N-Acetyl-D-Glucosamin-Gruppen eine lineare Struktur. Durch biochemische Modifikation wird Chitin teilweise deacetyliert und in Chitosan transformiert. Aus diesem kann dann enzymatisch oder durch saure Hydrolyse D-Glucosamin erhalten werden. D-Glucosamin kann auch direkt aus Chitin durch Erhitzen in verdünnter Salzsäure erhalten werden.

Als Extrakt der Muschel *Perna canaliculus* wird ein Produkt verwendet, welches aus dem Fleisch des Tieres oder aus den Organen erhältlich ist, insbesondere eines der folgenden Produkte:
a) ein durch Trocknen und Mahlen des gesamten Fleisches der Muschel erhältliches Pulver;
b) eine durch Lösemittelextraktion des Muschelfleisches oder des unter a) erwähnten Trockenpulvers erhältliche Lipidextraktionsmischung;
c) ein durch Trocknen und Mahlen der Gonaden der Muschel erhältliches Pulver; oder
d) eine durch Lösemittelextraktion der Gonaden oder des unter c) erwähnten Trockenpulvers erhältliche Lipidextraktionsmischung.

Die Herstellung dieser Extrakte kann wie folgt durchgeführt werden:
Extrakt a): Das Äußere der Muschel wird durch Besprengen unter hohem Druck gewaschen, und das Fleisch wird hiernach mechanisch aus der Schale entfernt, wobei sichergestellt wird, daß keine Wärme angewendet wird, so daß die Temperatur 10°C nicht überschreitet. Anschließend wird das Produkt auf Bakterien- und Schwermetallgehalt geprüft, wobei solche Muscheln verworfen werden, die derartige Verunreinigungen aufweisen. Das Fleisch wird danach in eine Vermahlungsmaschine eingebracht und in kleine Stücke pulverisiert, die dann auf ein Tablet in vorzugsweise einer gleichförmigen Dicke von etwa 1,9 cm gegeben und anschließend gefriergetrocknet werden. Das gefriergetrocknete Material wird dann zu einem feinen Pulver zerrieben und dicht verpackt, vorzugsweise in unter Vakuum verpackte Behälter.
Extrakt c): Zur Herstellung eines Pulvers gemäß c) kann die gleiche Vorgehensweise wie unter a) verwendet werden, nur daß die Gonaden mechanisch von dem Fleisch getrennt werden, bevor sie zermahlen werden.

Die Lipidextraktionsmischungen b) und d) können von dem gesamten Fleisch oder den Gonaden der Muschel durch Extraktion mit einem geeigneten organischen Lösungsmittel, welches Lipide lösen kann, z.B. mit halogenierten aliphatischen Kohlenwasserstoffen, wie z.B. Methylenchlorid, Ethylenchlorid, Chloroform, oder Tetrachlorkohlenstoff, z.B. mit aromatischen Kohlenwasserstoffen, wie z.B. Benzol, Toluol oder Xylenen, oder z.B. mit Estern wie z.B. Ethylacetat erhalten werden. Die Extraktion wird gemäß dem Fachmann bekannten Verfahren durchgeführt. Die auf diese Art und Weise erhaltenen Lipidextraktionsmischungen können direkt zum Zermahlen verwendet werden, wenn der organische Lösemittelextrakt vor dem Trocknen ausreichend getrocknet wurde. Alternativ können die Trockenextrakte a) und c) zur Extraktion mit dem organischen Lösungsmittel verwendet werden.

Die Zusammensetzung des Muschelextrakts gemäß c) ist wie folgt:(alle Angaben beziehen sich auf das Gewicht):
- Feuchtigkeitsgehalt:: 0,65 % bis 3,21 %,
durchschnittlich 2,23 %;
- Lipidgehalt:: 0,67 % bis 10,54 %,
durchschnittlich 9,09 %;
- Proteingehalt:: 52,13 % bis 55,60 %,
durchschnittlich 53,57 %;
- Kohlenhydratgehalt:: 18,60 % bis 24,29 %,
durchschnittlich 22,25 %;
- Aschegehalt:: 11,7 % bis 14,9 %,
durchschnittlich 12,83 %;

Die Erfindung betrifft insbesondere Diätetika und pharmazeutische Präparate umfassend die erfindungsgemäße Zusammensetzung. Die erfindungsgemäßen diätetischen und pharmazeutischen Präparate umfassen neben der erfindungsgemäßen Zusammensetzung pharmazeutisch bzw. ernährungsphysiologisch annehmbare Trägerstoffe. Diese Trägerstoffe hängen sowohl von der Verabreichungsform wie auch von der zu behandelnden Spezies ab. Beispielsweise können Getreide, Bierhefen, Malz, Gemüsearten und Kräuter für erfindungsgemäße diätetische und pharmazeutische Präparate, die Tieren oral verabreicht werden, verwendet werden. Die Wahl der Trägerstoffe in Abhängigkeit der Verabreichungsform bzw. der zu behandelnden Spezies sind dem Fachmann bekannt.

Das erfindungsgemäße diätetische und pharmazeutische Präparat enthält vorzugsweise 0,5 bis 35 Gew.%, besonders bevorzugt 1 bis 20 Gew.%, am bevorzugtesten 2,5 bis 10 Gew.% der Glucosamin-haltigen Komponente. Der Extrakt von *Perna canaliculus* ist vorzugsweise in einer Menge von 0,5 bis 40 Gew.%, besonders bevorzugt 1 bis 20 Gew.% , am bevorzugtesten 2 bis 12 Gew.% in dem erfindungagemäßen Präparat enthalten.

Weiterhin stellt die Erfindung die Verwendung der erfindungsgemäßen Zusammensetzung zur nutritiven Versorgung des Gelenkknorpels und zur Behandlung von Osteoarthritis bei Menschen und Tieren bereit. Bei der Behandlung von Menschen ist insbesondere eine Dosierung von 10 bis 20 mg Glucosamin pro kg Körpergewicht und pro Tag und 18 mg Muschelextrakt pro kg Körpergewicht und pro Tag vorteilhaft. Es sind jedoch auch Dosiserhöhungen bis zu der 2- bis 4-fachen Menge möglich.

Bei der Behandlung von Tieren hängt die Dosis von der zu behandelnden Spezies ab. Bei Hunden hat sich eine Dosierung von 16 bis 40 mg Glucosamin pro kg Körpergewicht pro Tag und 11 bis 28 mg Muschelextrakt pro kg Körpergewicht pro Tag als vorteilhaft erwiesen. Bei der Behandlung von Pferden ist eine Dosierung von 5,4 bis 7,2 mg Glucosamin pro kg Körpergewicht pro Tag und und 6,7 bis 9,0 mg Muschelextrakt pro kg Körpergewicht pro Tag bevorzugt. Die Dosis kann jedoch aufgrund der nicht vorhandenen Toxizität und guten Verträglichkeit der Inhaltsstoffe der erfindungsgemäßen Zusammensetzung ohne weiteres verdoppelt oder sogar verdreifacht werden. Eine weitere Dosissteigerung, obwohl unschädlich für den Organismus, ist jedoch nicht bevorzugt, da sie keinen weiteren physiologischen Vorteil bietet und lediglich zu höheren Kosten führt.

Die oben angegebenen Dosierungen können in einer oder in mehreren Portionen verabreicht werden. Eine Portion kann jeweils aus einer oder mehreren Verabreichungseinheiten wie z.B. Pellets, Tabletten, Kapseln oder Granulaten bestehen. Im folgenden werden diesbezüglich einige Beispiele aufgeführt werden.

### Beispiel 1:

Herstellung eines Präparates für Hunde (2,85 Gew.% Glucosamin-Komponente/2,0 Gew.% Muschelextrakt) in Form von Pellets: Die von der Qualitätskontrolle freigegebenen Rohstoffe werden gemäß Herstellungsformel eingewogen. Es kommen nachfolgende Rohstoffe zum Einsatz: Glucosamin-Komponente, Muschelextrakt, Fermentgetreide, fermentierte Bierhefe, Maiskeime, Leinöl, Kräuter. In einem geeigneten Freifallmischer werden diese Rohstoffe ausreichend lange durchmischt bis die Wirkstoffe mit den Trägerstoffen homogen vermischt sind. Anschließend wird die Mischung in einer Würfelpresse zu einer zylindrischen Form mit 4 mm Durchmesser und 0,8 cm bis 1,2 cm Länge, dem sog. Pellet, gepreßt. Es wird beim Pressen sichergestellt, daß 37° C im Pellet und in der Umgebung niemals überschritten wird.

### Beispiel 2:

Eine Zusammensetzung wurde in der gleichen Art und Weise wie in Beispiel 1 hergestellt, nur daß 5,7 Gew.% der glucosaminhaltigen Komponente und 4.0 Gew.% Muschelextrakt verwendet wurden. Diese Zusammensetzung eignet sich in besonderem Maße zur Behandlung von Hunden.

### Beispiel 3:

Herstellung eines Präparates für Pferde (4,5 Gew.% Glucosamin-Komponente/5,6 Gew.% Muschelextrakt) in Form von Pellets:

Die von der Qualitätskontrolle freigegebenen Rohstoffe werden gemäß Herstellungsformel eingewogen. Es kommen nachfolgende Rohstoffe zum Einsatz: Glucosamin-Komponente, Muschelextrakt, Fermentgetreide, fermentierte Bierhefe, Maiskeime, Sojaöl, Kräuter. In einem geeigneten Freifallmischer werden diese Rohstoffe ausreichend lange durchmischt bis die Wirkstoffe mit den Trägerstoffen homogen vermischt sind. Anschließend wird die Mischung in einer Würfelpressen zu einer zylindrischen Form mit 4 mm Durchmesser und 1,2 cm bis 1,6 cm Länge, dem sog. Pellet, gepreßt. Es wird beim Pressen sichergestellt, daß 37° C im Pellet und in der Umgebung niemals überschritten wird.

### Beispiel 4:

Eine Zusammensetzung wurde in der gleichen Art und Weise wie in Beispiel 3 hergestellt, nur daß 9,0 Gew.% der glucosaminhaltigen Komponente und 11,2 Gew.% Muschelextrakt verwendet wurden. Diese Zusammensetzung eignet sich in besonderem Maße zur Behandlung von Pferden.

### Beispiel 5:

Herstellung eines Präparates für Menschen (4,7% Glucosamin-Komponente/5,4% Muschelextrakt) in Granulatform: Die von der Qualitätskontrolle freigegebenen Rohstoffe werden gemäß Herstellungsformel eingewogen. Es kommen nachfolgende Rohstoffe zum Einsatz: Glucosamin-Komponente, Muschelextrakt, Süßmolke, Kartoffelstärke, Sorbitol, Weizen- und Haferrohfaser, Erbsenrohfaser, Pflanzenfett, Malzextrakt, Aromen, Süßstoff Aspartam. In einem geeigneten Freifallmischer werden diese Rohstoffe ausreichend lange durchmischt bis die Wirkstoffe mit den Trägerstoffen homogen vermischt sind. Anschließend wird die Mischung mittels eines Kompaktors trockengranuliert. Nach Siebung des Granulates auf 1,5 mm wird nochmals 20 Minuten gemischt.

## Patentansprüche

1. Zusammensetzung, umfassend einen Extrakt der Muschel *Perna canaliculus* und eine Glucosamin-Komponente.

2. Zusammensetzung gemäß Anspruch 1, dadurch gekennzeichnet, daß das Gewichtsverhältnis von Glucosamin-Komponente zu dem Extrakt in dem Bereich von 0,2 bis 6 liegt.

3. Zusammensetzung gemäß Anspruch 2, dadurch gekennzeichnet, daß das Gewichtsverhältnis von Glucosamin-Komponente zu dem Extrakt in dem Bereich von 0,5 bis 2,5 liegt.

4. Zusammensetzung gemäß einem der vorhergehenden Ansprüche, dadurch gekennzeichnet, daß die Glucosamin-Komponente D-Glucosamin, D-Glucosaminsulfat, D-Glucosamin-HCl N-Acetyl-D-Glucosamin oder eine Mischung hiervon ist.

5. Zusammensetzung gemäß einem der vorhergehenden Ansprüche, dadurch gekennzeichnet, daß die Glucosamin-Komponente durch Hydrolyse von Chitin erhältlich ist.

6. Zusammensetzung gemäß einem der vorhergehenden Ansprüche, dadurch gekennzeichnet, daß der Extrakt ein Pulver ist, welches durch Trocknen und Mahlen der Gonaden der Muschel *Perna canaliculus* erhältlich ist.

7. Zusammensetzung gemäß einem der vorhergehenden Ansprüche, dadurch gekennzeichnet, daß der Extrakt einen Flüssigkeitsgehalt von 0,65 bis 3,21 Gew.%; einen Lipidgehalt von 0,67 bis 10,54 Gew.%; einen Proteingehalt von 52,13 bis 55,60 Gew.%; einen Kohlenhydratgehalt von 18,60 bis 24,29 Gew.%; und einen Aschegehalt von 11,7 bis 14,9 Gew.% aufweist.

8. Pharmazeutisches Präparat, umfassend eine Zusammensetzung gemäß einem oder mehreren der Ansprüche 1 bis 7 und pharmazeutisch annehmbare Trägerstoffe.

9. Pharmazeutisches Präparat gemäß Anspruch 8, dadurch gekennzeichnet, daß die Glucosamin-Komponente zu 0.5 bis 35 Gew.%, vorzugsweise zu 1 bis 20 Gew.%, in dem Präparat enthalten ist.

10. Pharmazeutisches Präparat gemäß Anspruch 8 oder 9, dadurch gekennzeichnet, daß der Extrakt von *Perna canaliculus* in einer Menge von 0,5 bis 40 Gew.%, vorzugsweise 1 bis 20 Gew.%, in dem Präparat enthalten ist.

11. Verwendung der Zusammensetzung gemäß einem oder mehreren der Ansprüche 1 bis 7 zur Behandlung von Osteoarthritis beim Menschen.

12. Verwendung der Zusammensetzung gemäß einem oder mehreren der Ansprüche 1 bis 7 zur Behandlung von Osteoarthritis beim Tier.

13. Verwendung der Zusammensetzung gemäß einem oder mehreren der Ansprüche 1 bis 7 zur nutritiven Versorgung des Gelenkknorpels beim Menschen.

14. Verwendung der Zusammensetzung gemäß einem oder mehreren der Ansprüche 1 bis 7 zur nutritiven Versorgung des Gelenkknorpels beim Tier.
